# EUROPÄISCHE PATENTSCHRIFT

(11) **EP 1 430 844 B1**
(45) Veröffentlichungstag und Bekanntmachung des Hinweises auf die Patenterteilung: **01.03.2006**
(21) Anmeldenummer: 03024492.5
(22) Anmeldetag: 24.10.2003
(51) Int. Cl.: A61B 17/17

(54) **Zentrierhilfe für ein Gelenkkopf-Kappenimplantat eines künstlichen Hüftgelenkes**
Centering aid for a joint head cap implant of an artificial hip joint
Moyen d'aide au centrage pour un implant de calotte de tête articulaire d'une articulation artificielle de la hanche

(30) Priorität: 19.12.2002 DE 10261813
(43) Veröffentlichungstag der Anmeldung: 23.06.2004
(73) Patentinhaber: ESKA Implants GmbH & Co., 23556 Lübeck (DE)
(72) Erfinder: Grundei, Hans, Dr. Ing., 23558 Lübeck (DE); Karpf, Peter-Michael, Professor Dr., 84028 Landshut (DE)
(74) Vertreter: Fuchs Mehler Weiss & Fritzsche

(56) Entgegenhaltungen:
- DE-A- 19 547 426
- DE-B- 1 164 019
- US-A- 4 896 663
- US-A- 6 156 069

## Beschreibung

Die vorliegende Erfindung betrifft eine Zentrierhilfe für Gelenkkopf-Kappenimplantat eines künstlichen Hüftgelenkes.

In jüngster Zeit kommen verstärkt sogenannte Kappenimplantate zur Anwendung, welche über den präparierten natürlichen Restgelenkkopf gesetzt werden und in dieser Lage dann fixiert werden. Voraussetzung für den Einsatz eines solchen Kappenimplantates ist die sorgfältige Vorbereitung des natürlichen Restgelenkkopfes, damit dann eine möglichst weitgehende Übereinstimmung zwischen der Kopfbelastungsachse und der Achse der Kappe erzielt wird. Darüber hinaus sind der CCD- und der Antekurvationswinkel so einzustellen, dass diese weitgehend den Werten im natürlichen Hüftgelenk entsprechen.

Erschwerend kommt hinzu, dass sich beispielsweise bei einer Nekrose der Gelenkkopf verlagert. Meist ist dies in Richtung caudal der Fall. Aber auch nach medial oder lateral sind Verschiebungen durchaus möglich, abhängig vom jeweiligen Krankheitsbild. In einem solchen Falle das Kappenimplantat so einzustellen, dass sich praktisch natürliche Winkel- und Lageverhältnisse ergeben, ist relativ schwierig.

Bislang war dies erfahrenen Operateuren vorbehalten. Allerdings ist die Zahl von Fehlstellungen des Kappenimplantates nach erfolgter Implantation nicht vernachlässigbar klein.

Aus den Druckschriften DE 1 164 019 B und US-A-6 156 069 sind Lehren zur Anpassung eines Hüftgelenkkopfes vor der Implantation eines Hüftgelenkimplantates bekannt. Hier werden nach strengen Regeln Festlegungen betrieben, die in keiner Weise patientenindividuell sein können.

Vor diesem Hintergrund ist es die Aufgabe der vorliegenden Erfindung, eine Hilfseinrichtung anzugeben, mit welcher die Resektion des natürlichen Hüftgelenkkopfes so vorbereitet werden kann, dass die natürlichen, patientenindividuellen Stellungen des natürlichen Hüftgelenkkopfes übertragen werden auf das Kappenimplantat.

Gelöst wird diese Aufgabe durch eine Zentrierhilfe mit den Merkmalen des Anspruchs 1. Vorteilhafte Weiterbildungen sind in den Unteransprüchen angegeben.

Demnach weist die Zentrierhilfe eine auf den Restgelenkkopf des natürlichen Hüftgelenkes setzbare kalottenförmige Kappe mit einer radialen Durchbohrung in ihrem Polbereich und wenigstens einer weiteren Durchbohrung, deren Achsen parallel zu der Schenkelhalsachse verlaufen, eine Lehre von blockförmiger Gestalt mit einer Anzahl von Durchbohrungen, deren Mittelpunkte auf einer Gerade liegen und deren Achsen mit der Schenkelhalsachse fluchten und mit einem Arretierungszapfen, welcher in eine in dem Restgelenkkopf eingebrachte Bohrung setzbar ist, und wenigstens zwei Führungsstifte auf, welche sowohl durch die Durchbohrung im Polbereich als auch durch die wenigstens eine weitere Durchbohrung setzbar und in dem Restgelenkkopf fixierbar sind.

Zu bemerken ist, dass es sich vorliegend um eine Zentrierhilfe, nicht um eine Zentrierlehre handelt. Die erfindungsgemäße Zentrierhilfe erleichtert das Auffinden des anatomischen Pol-Zentrums des Hüftgelenkkopfes. Hierdurch wird ein spannungsfreies Zusammenwirken von Hüfte und Becken gewährleistet.

Zum Auffinden der zutreffenden Lage geht der Operateur wie folgt durch: Zunächst markiert er die Basiskante des natürlichen Hüftgelenkkopfes, also den Übergang zum Schenkelhals, beispielsweise mittels eines geeigneten Markierungsstiftes und setzt dann die kalottenförmige Kappe auf den natürlichen Gelenkkopf, nachdem er die Größe des natürlichen Gelenkkopfes ermittelt hat und eine entsprechend große Kappe ausgewählt hat. Der Operateur ertastet nun unter Beobachtung der gekennzeichneten Basis des natürlichen Gelenkkopfes die zutreffende Lage der aufgesetzten Kappe. Diese ist dann gegeben, wenn eine möglichst große Übereinstimmung zwischen der erwähnten Basiskante und dem Äquator der Kappe erzielt worden ist. Sodann wird der wenigstens eine Führungsstift durch die Durchbohrung im Polbereich der Kappe gesetzt und in den natürlichen Gelenkkopf getrieben, beispielsweise mittels eines Hammers. Dann zieht der Operateur die Kappe von dem natürlichen Gelenkkopf ab, wobei der Führungsstift im Gelenkkopf verbleibt.

Sodann erfolgt der Einsatz der Lehre mit der Anzahl von Durchbohrungen. Eine Durchbohrung entspricht der Nulllage, also der Istlage des Pols des gegenüber dem gesunden Zustand lageversetzten Gelenkkopfes. Die Lehre wird auf den im Gelenkkopf verbliebenen Führungsstift gesetzt und zwar so, dass sie mit der Bohrung entsprechend der Nulllage auf den Führungsstift aufgefädelt wird. Die Lehre weist einen Arretierungszapfen auf, der in eine in den Gelenkkopf eingebrachte Bohrung gesetzt wird, so dass die Lehre lagestabil auf dem Gelenkkopf verharrt.

Durch die Ausrichtung der wenigstens einen weiteren Durchbohrung in der Kappe zu der Durchbohrung im Polbereich zu der Schenkelhalsachse einerseits sowie durch die Ausrichtung der Bohrungen in der Lehre ebenfalls in Richtung der Schenkelhalsachse ergibt sich nun die Möglichkeit, eine Korrektur vorzunehmen, die darauf abzielt, die Verlagerung des natürlichen Gelenkkopfes, meist nach caudal, zu kompensieren. Es wird nämlich eine gewissermaßen Parallelverschiebung der ermittelten Polachse in Richtung auf die Schenkelhalsachse durchgeführt, und zwar unter Zuhilfenahme der Durchbohrungen in der Lehre, deren Mittelpunkte - auf einer Gerade liegen und deren Achsen mit der Schenkelhalsachse fluchtet. Der Operateur hat nun zu entscheiden, in welche der Bohrungen der Lehre der zweite Führungsstift gesetzt wird. Hier kommt es wieder auf die Erfahrung des Operateurs an. Eine mathematisch exakte Lösung gibt es hierfür nicht. So wird der Operateur die Durchbohrung in der Lehre wählen, die nach seinem (optischen) Eindruck weitestgehend mit der Zentralachse des Schenkelhalses fluchtet und den zweiten Führungsstift durch diese Durchbohrung setzen und diesen mittels eines Werkzeuges in den Restgelenkkopf schlagen.

Der zweite Führungsstift dient danach als Auffädelhilfe für einen Bohrer, der eine Bohrung in den Gelenkkopf einbringt. Mittels eines Fräsers wird dann der Gelenkkopf beispielsweise zu einem zylindrischen Stumpf gefräst, auf den das entsprechend komplementär ausgebildete Kappenimplantat gesetzt wird, wobei in die verbliebene Bohrung das Stielteil des Kappenimplantates greift. Ein derartiges Kappenimplantat ist im Übrigen bekannt aus der DE-A-102 18 801. Die Lageverhältnisse sind nun weitgehend so, wie sie im natürlichen gesunden Gelenk vor der Erkrankung gewesen waren, wodurch das spannungsfreie Muskelspiel von Hüfte und Becken gewährleistet wird.

Gemäß einer weiteren vorteilhaften Weiterbildung ist der Polbereich der Kappe von einer zylindrischen Muffe eingefasst, wobei diese Muffe eine die Durchbohrung im Polbereich der Kappe fortsetzende Durchbohrung aufweist. Durch diese Maßnahme wird die Führung der Kappe auf dem Führungsstift verbessert und die Übertragung der natürlichen Winkelverhältnisse auf das Kappenimplantat wird noch genauer aufgrund der exakteren Führung.

Gemäß einer noch vorteilhaften Weiterbildung ist vorgesehen, dass an der Außenseite der Kappe ein Ansatz vorgesehen ist, mit dem eine Halterung lösbar verbindbar ist. Diese Halterung dient der einfacheren Handhabung der Kappe durch den Operateur und kann wahlweise mit der Kappe verbunden werden.

Die Erfindung wird anhand eines Ausführungsbeispiels gemäß den Zeichnungsfiguren näher erläutert. Hierbei zeigt:
- Fig. 1: Einzelteile der Zentrierhilfe,
- Fig. 2: die Aufsicht auf den Polbereich der Kappe der Zentrierhilfe,
- Fig. 3: schematisch die Anwendung der Zentrierhilfe,
- Fig. 4: schematisch den Einsatz der Lehre.

Nachfolgend sind gleiche Teile mit denselben Bezugszeichen versehen.

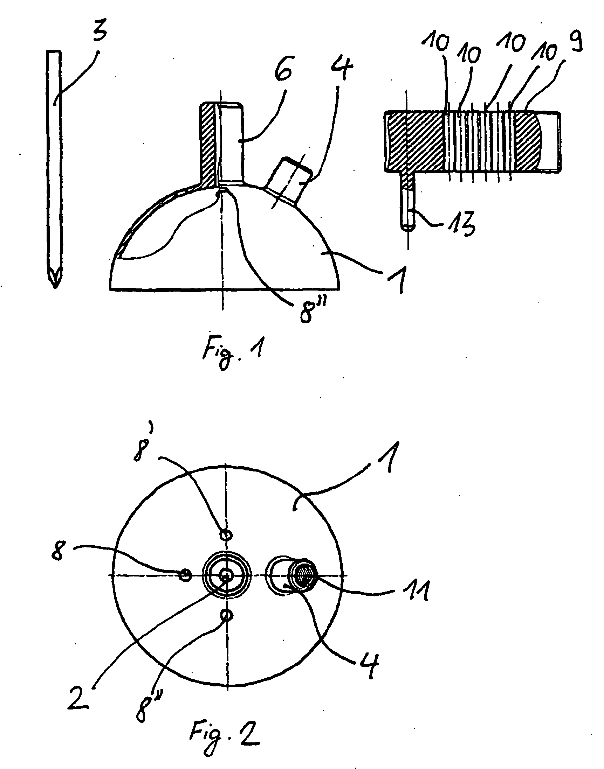

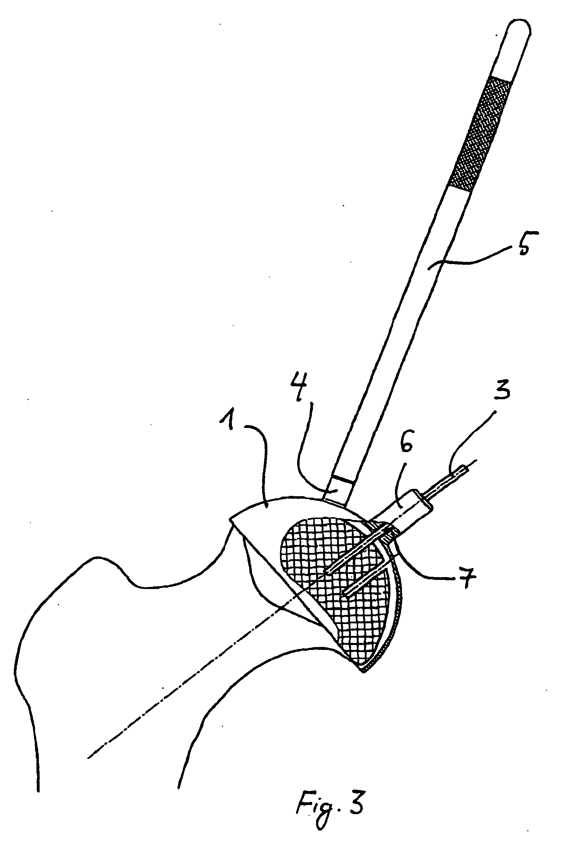

Einen ersten Überblick verschafft Fig. 1. Darin abgebildet ist eine kalottenförmige Kappe 1. Der Polbereich der Kappe 1 ist von der zylindrischen Muffe 6 eingefasst, die eine bis zum Polbereich der Kappe 1 reichende Durchbohrung 7 aufweist (Fig. 3). Erkennbar ist darüber hinaus eine weitere radiale Durchbohrung 8 in der Kappe. Die mindestens eine weitere Durchbohrung 8 ist so angeordnet, dass sie auf einer Achse mit der Durchbohrung 2 im Polbereich der Kappe 1 liegt, welche für den Operateur (optisch) der Schenkelhalsachse entspricht. Dies ist für die spätere Parallelverschiebung der Polachse von entscheidender Bedeutung, da in den meisten Fällen die Polachse des natürlichen Gelenkkopfes bei einer Nekrose nach caudal abwandert. Im vorliegenden Falle weist die Kappe 1 neben der Durchbohrung 2 im Polbereich und der Durchbohrung 8 zwei weitere Durchbohrungen 8', 8" auf (Fig. 2). Die weiteren Durchbohrungen 8' und 8" sind vorgesehen, um etwaige Abwanderungen des Gelenkkopfes in Richtung lateral oder medial kompensieren zu können. Die Kappe 1 weist darüber hinaus einen Ansatz 4 mit einem Innengewinde 11 auf (Fig. 2). Mit diesem Innengewinde 11 ist eine Halterung 5 (Fig. 3) verschraubbar, welche dem Operateur die notwendige Handhabe bietet, um die Kappe 1 auf dem Gelenkkopf möglichst exakt zu positionieren.

Durch die Durchbohrung 7 in der zylindrischen Muffe 6 ist ein Führungsstift 3 setzbar, und zwar so, dass der Führungsstift 3 im Restgelenkkopf fixiert wird. Aus diesem Grunde ist das untere Ende des Führungsstiftes vorliegend nagelförmig ausgebildet. Dabei achtet der Operateur darauf, dass der Führungsstift (optisch) mit der Schenkelhalsachse fluchtet.

Zur Vorbereitung des Gelenkkopfes auf die notwendig werdende Resektion wird die Kappe 1 nach Vorauswahl einer geeigneten Größe auf den Gelenkkopf gesetzt. Der Operateur ertastet sodann die zutreffende Lage der Kappe auf dem Gelenkkopf. Hierzu erleichtert die Halterung 5 die Lageverstellung. Die zutreffende Lage ist dann gegeben, wenn die Basiskante des natürlichen Gelenkkopfes und Äquator der Kappe 1 möglichst deckungsgleich sind. Sodann wird der Operateur einen ersten Führungsstift 3 in die Durchbohrung 7 in der Muffe 6 setzen und ihn in den Gelenkkopf schlagen.

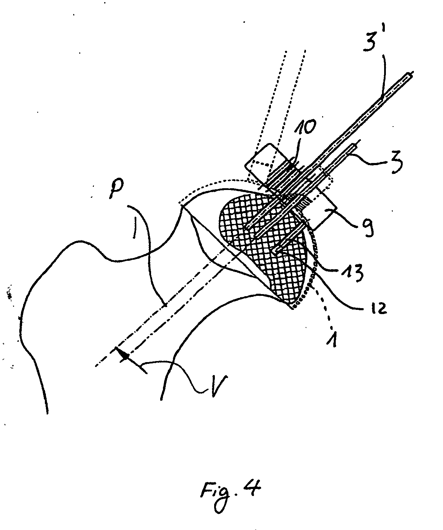

Danach wird die Kappe 1 vom Gelenkkopf abgezogen, wobei jedoch der Führungsstift 3 in seiner Lage im Gelenkkopf verbleibt. Nun kommt die Lehre 9 zum Einsatz, und zwar zur Parallelverschiebung der ermittelten Polachse des erkrankten Gelenkkopfes hin zu der Polachse, die in etwa der Polachse des natürlichen Gelenkkopfes entspricht. Hierzu wird auf die Fig. 4 verwiesen.

In Phantomlinien ist in Fig. 4 die dann in diesem Zustand bereits entfemte Kappe 1 angedeutet. Die Lehre 9 ist mit ihrem Arretierungszapfen 13 in einer Bohrung im erkrankten Gelenkkopf arretiert. Die Lehre 9 ist auf den im Gelenkkopf verbliebenen Führungsstift 3 mit einer Durchbohrung aufgefädelt, welche der Nulllage entspricht, das heißt entsprechend der Polachse des erkrankten Gelenkkopfes. Die weiteren Maßnahmen dienen zur Parallelverschiebung dieser Polachse hin zu einer Polachse, welche weitgehend der Polachse des Gelenkkopfes vor der Erkrankung entspricht. Diese Parallelverschiebung ist durch den Pfeil V mit Richtungsangabe angedeutet. Der Operateur wählt für einen zweiten Führungsstift 3' nun die Durchbohrung 10 in der Lehre 9 aus, deren Achse (optisch) mit der Schenkelhalsachse P übereinstimmt. Hier ist nochmals zu betonen, dass es sich bei der Zentrierhilfe nicht um ein mathematisch exaktes Gerät handelt, sondern vielmehr um eine Positionierhilfe für den Operateur. Nach dem Fixieren des Führungsstiftes 3' im Gelenkkopf wird die Lehre 9 abgezogen und der Führungsstift 3', der im Gelenkkopf verbleibt, dient als Auffädelhilfe für weitere Werkzeuge, beispielsweise für Bohrer und Fräser.

## Patentansprüche

1. Zentrierhilfe für ein Gelenkkopf-Kappenimplantat eines künstlichen Hüftgelenkes, aufweisend
- eine auf den Restgelenkkopf des natürlichen Hüftgelenkes setzbare kalottenförmige Kappe (1) mit einer radialen Durchbohrung (2) in ihrem Polbereich und wenigstens einer weiteren Durchbohrung (8), deren Achsen parallel zu der Schenkelhalsachse (P) verlaufen,
- eine Lehre (9) von blockförmiger Gestalt mit einer Anzahl von Durchbohrungen (10), deren Mittelpunkte auf einer Gerade liegen und deren Achsen mit der Schenkelhalsachse (P) fluchten und mit einem Arretierungszapfen (13), welcher in eine in den Restgelenkkopf eingebrachte Bohrung (12) setzbar ist, und
- wenigstens zwei Führungsstifte (3, 3'), welche sowohl durch die Durchbohrung (2) im Polbereich als auch durch eine der Durchbohrungen (10) der Lehre setzbar und in dem Restgelenkkopf fixierbar ist.

2. Zentrierhilfe nach Anspruch 1, bei der der Polbereich der Kappe (1) von einer zylindrischen Muffe (6) mit einer die Durchbohrung (2) fortsetzenden Durchbohrung (7) eingefasst ist.

3. Zentrierhilfe nach Anspruch 1 oder 2, bei der an der Außenseite der Kappe (1) ein Ansatz (4) vorgesehen ist, mit dem eine Halterung (5) lösbar verbindbar ist.

## Claims

1. Centering aid for a joint head cap implant of an artificial hip joint having
- a dome-shaped cap (1), which can be placed on the residual joint head of the natural hip joint, having a radial perforation (2) in its pole region and at least one further perforation (8), the axes of which run parallel to the femoral neck axis (P),
- a template (9) of block-like form having a number of perforations (10), the central points of which lie on a straight line and the axes of which are aligned with the femoral neck axis (P) and having a locking pin (13), which can be placed in a bore (12) introduced into the residual joint head, and
- at least two guide pins (3, 3'), which can be placed through the perforation (2) in the pole region and through one of the perforations (10) of the template and can be fixed in the residual joint head.

2. Centering aid according to claim 1, in which the pole region of the cap (1) is enclosed by a cylindrical sleeve (6) having a perforation (7) continuing the perforation (2).

3. Centering aid according to claim 1 or 2, in which an attachment (4), to which a holder (5) can be releasably connected, is provided on the outer side of the cap (1).

## Revendications

1. Moyen d'aide au centrage pour un implant de calotte de tête articulaire d'une articulation artificielle de la hanche, présentant :
- une calotte (1) en forme de dôme pouvant être posée sur la tête articulaire restante de l'articulation naturelle de la hanche, avec un alésage traversant radial (2) dans sa partie polaire et au moins un autre alésage traversant (8) dont les axes s'étendent parallèlement à l'axe du col du fémur (P),
- un gabarit (9) de configuration en forme de bloc avec un certain nombre d'alésages traversants (10), dont les centres se situent sur une droite et dont les axes sont alignés avec l'axe du col du fémur (P) et avec un tourillon d'arrêt (13) qui peut être placé dans un alésage (12) monté dans la tête articulaire restante, et
- au moins deux tiges de guidage (3, 3') qui peuvent être placées à la fois à travers l'alésage traversant (2) dans la région polaire et aussi à travers l'un des alésages traversants (10) et qui peuvent être fixées dans la tête articulaire restante.

2. Moyen d'aide au centrage selon la revendication 1, dans lequel la région polaire de la calotte (1) est enchâssée par une gaine cylindrique (6) avec un alésage traversant (7) continuant l'alésage traversant (2).

3. Moyen d'aide au centrage selon la revendication 1 ou 2, dans lequel sur le côté extérieur de la calotte (1) on prévoit un insert (4) auquel une fixation (5) peut être assemblée de manière détachable.
